(19) Europäisches Patentamt

European Patent Office

Office européen des brevets

(11) **EP 1 719 447 A1**

(12) **EUROPÄISCHE PATENTANMELDUNG**

(43) Veröffentlichungstag:
**08.11.2006 Patentblatt 2006/45**

(51) Int Cl.:
***A61B 5/00*** *(2006.01)*

(21) Anmeldenummer: **05009986.0**

(22) Anmeldetag: **07.05.2005**

(84) Benannte Vertragsstaaten:
**AT BE BG CH CY CZ DE DK EE ES FI FR GB GR HU IE IS IT LI LT LU MC NL PL PT RO SE SI SK TR**
Benannte Erstreckungsstaaten:
**AL BA HR LV MK YU**

(71) Anmelder:
• **F. HOFFMANN-LA ROCHE AG**
**4070 Basel (CH)**
Benannte Vertragsstaaten:
**AT BE BG CH CY CZ DK EE ES FI FR GB GR HU IE IS IT LI LT LU MC NL PL PT RO SE SI SK TR**
• **Roche Diagnostics GmbH**
**68305 Mannheim (DE)**
Benannte Vertragsstaaten:
**DE**

(72) Erfinder:
• **Ferrari, Stefano**
**68161 Mannhein (DE)**
• **Quarder, Ortrud**
**69115 Heidelberg (DE)**
• **Stephan, Peter**
**68167 Mannheim (DE)**

(74) Vertreter: **Pfiz, Thomas et al**
**Patentanwälte Wolf & Lutz**
**Hauptmannsreute 93**
**70193 Stuttgart (DE)**

(54) **Verfahren und Vorrichtung zur Bestimmung der Glucose-Konzentration in Gewebflüssigkeit**

(57) Die Erfindung betrifft ein Verfahren und eine Vorrichtung zur Bestimmung der Glucose-Konzentration in Gewebeflüssigkeit (40), womit in einer durch Mikrodialyse, Mikroperfusion oder Ultrafiltration (Sonde 10) erhaltenen Probenflüssigkeit (42) Messwerte für Glucose und für eine endogene Referenzsubstanz erfasst werden (Sensor 12) und der Glucosewert nach Maßgabe des Messwerts für die Referenzsubstanz korrigiert wird. Hier wird vorgeschlagen, dass die Wiederfindungsrate für Glucose aus einer nichtlinearen Beziehung zu der Wiederfindungsrate für die ionische Referenzsubstanz bestimmt und der Glucose-Messwert damit korrigiert wird, und dass zudem die Konzentration von Lactat und/oder Pyruvat als weitere Referenzsubstanz in der Probenflüssigkeit zur weiteren Korrektur herangezogen wird.

Fig.2

EP 1 719 447 A1

**Beschreibung**

Die Erfindung betrifft ein Verfahren zur Bestimmung der Glucose-Konzentration in Gewebeflüssigkeit bei welchem in einer durch Mikrodialyse, Mikroperfusion oder Ultrafiltration aus einem Körpergewebe erhaltenen Probenflüssigkeit Messwerte für Glucose und für eine endogene Referenzsubstanz erfasst werden und der Messwert für Glucose nach Maßgabe des Messwerts für die Referenzsubstanz korrigiert wird. Die Erfindung betrifft weiter eine entsprechende Vorrichtung.

Körpergewebe bestehen aus Zellen in einer Flüssigkeitsumgebung, in der Stoffwechselprodukte zwischen den Zellen und den Blutgefäßen transportiert werden. Für eine Glucose-Überwachung speziell von Diabetes-Patienten kann eine Sonde längere Zeiträume in Gewebe eingesetzt werden, um über Diffusionsvorgänge Inhaltsstoffe aus der Gewebeflüssigkeit kontinuierlich zu gewinnen und aus dem Effusat den Glucosegehalt im Gewebe zu bestimmen. Dieser kann eng mit dem Blutglucosegehalt korreliert werden, ohne dass ein invasiver Zugang zum Blutkreislauf erforderlich wäre. Die Messung kann außerhalb des Körpers erfolgen, wobei ein Sensor mit der Probenflüssigkeit beaufschlagt wird. In diesem Zusammenhang ist eine ionische Referenztechnik bekannt, bei der durch eine ionenselektive Elektrode simultan zur Glucose auch ein ionischer Referenzwert, insbesondere $Na^+$ erfasst wird, um die Wiederfindung der Glucose im Dialysat zu kalibrieren. Hierbei wird davon ausgegangen, dass die bekannte Konzentration der Referenzsubstanz in der Körperflüssigkeit im Wesentlichen invariant ist. Die bekannten Auswerteverfahren setzen voraus, dass ein streng linearer Zusammenhang zwischen der Wiederfindung von endogenem Kalibrator und Glucose in der Probenflüssigkeit besteht. Empirische Vergleichsmessungen lassen daran jedoch zweifeln.

Ausgehend hiervon liegt der Erfindung die Aufgabe zugrunde, die im Stand der Technik aufgetretenen Nachteile zu vermeiden und eine Referenztechnik anzugeben, womit die tatsächlichen Körperwerte exakter bestimmbar und überprüfbar sind.

Zur Lösung dieser Aufgabe wird die in den unabhängigen Patentansprüchen angegebene Merkmalskombination vorgeschlagen. Vorteilhafte Ausgestaltungen und Weiterbildungen der Erfindung ergeben sich aus den abhängigen Ansprüchen.

Die Erfindung geht von dem Gedanken aus, sowohl lokale Effekte im Gewebe als auch Transportwiderstände zu berücksichtigen, wie sie aufgrund der speziellen Sonden bei der Mikrodialyse, Mikroperfusion oder Ultrafiltration auftreten. Dementsprechend wird gemäß einer ersten Erfindungsvariante vorgeschlagen, dass die Konzentration von Lactat und/oder Pyruvat als Referenzsubstanz in der Probenflüssigkeit bestimmt wird. Damit ist es möglich, die spezifischen Stoffwechselpfade beim Glucoseabbau heranzuziehen, um Rückschlüsse auf durch lokale Gewebereaktionen bedingte Effekte für die Messwertkorrektur zu treffen.

Vorteilhafterweise wird ein Konzentrationsverhältnis von Lactat zu Pyruvat in der Probenflüssigkeit zur Korrektur des Messwertes für Glucose gebildet. Eine vorteilhafte Ausgestaltung sieht vor, dass bei einem Konzentrationsverhältnis zwischen 10:1 und 20:1 eine lineare Korrektur in Abhängigkeit von dem Konzentrationsverhältnis durchgeführt wird, und ab einem Konzentrationsverhältnis von 20:1 der Messwert für Glucose durch eine Konstante korrigiert wird.

Gemäß einem weiteren Aspekt der Erfindung wird vorgeschlagen, dass die Wiederfindungsrate ($R_{GLU}$) für Glucose aus einer nichtlinearen Beziehung zu der Wiederfindungsrate ($R_{REF}$) für die ionische Referenzsubstanz bestimmt und der Glucose-Messwert damit korrigiert wird. Auf diese Weise kann eine Art technische Korrektur für Effekte, die hauptsächlich durch die spezifische Sondentechnik beispielsweise aufgrund von Membranvorgängen hervorgerufen werden, in spezifisch angepasster Form getroffen werden. Damit kann wesentlich genauer korrigiert werden als mit der herkömmlichen Linearbeziehung, welche als Winkelhalbierende im Diagramm der Wiederfindungsraten zwischen Glucose und Ionenreferenz verläuft. Dies kann insbesondere daran liegen, dass die Wiederfindung der Ionenreferenz durch die geringere Teilchengröße und/oder durch die Ladung vor allem bei rascher Perfusion höher ist als für Glucose. Die nichtlineare Kompensationskurve verläuft somit gekrümmt unterhalb der Winkelhalbierenden.

Gemäß einer besonders bevorzugten Ausgestaltung wird die Wiederfindungsrate ($R_{GLU}$) für Glucose gemäß der Beziehung

$$1 - R_{REF} = (1 - R_{GLU})^k$$

ermittelt, wobei k ein vorgegebener Wert ist. Dabei kann der Wert k als Verhältnis der Widerstände für den Übergang von Glucose und Referenzsubstanz zwischen der Gewebe- und der Probenflüssigkeit vorzugsweise empirisch bestimmt werden. Weiterhin wird vorgeschlagen, dass die Wiederfindungsrate ($R_{REF}$) für die Referenzsubstanz als Verhältnis aus dem Messwert für die Referenzsubstanz in der Probenflüssigkeit und einem konstanten Konzentrationswert in der Gewebeflüssigkeit ermittelt wird. Vorteilhafterweise wird Natrium als körpereigene ionische Referenzsubstanz herangezogen.

Eine besonders effektive und genaue Kompensation lässt sich dadurch erreichen, dass Natrium als Referenzsubstanz für eine Korrektur von messtechnischen Abweichungen und Lactat und/oder Pyruvat als Referenzsubstanz für eine Korrektur von lokal bedingten Abweichungen des Messwerts für Glucose in der Probenflüssigkeit von der tatsächlichen Glucose-Konzentration im Gewebe des Organismus herangezogen wird.

Die vorstehend beschriebene Kompensation lässt sich besonders vorteilhaft anwenden bei Techniken, bei denen die Probenflüssigkeit mittels einer im Gewebe befindlichen Sonde durch Perfusion mit Spülflüssigkeit oder durch Anlegen eines Unterdrucks gewonnen wird.

Eine zusätzliche Funktionalität wird dadurch erreicht, dass bei Überschreiten einer oberen und/oder unteren vorgegebenen Schwelle des Messwerts für die Referenzsubstanz eine Fehlfunktion signalisiert wird.

Die vorstehend in verfahrensmäßigem Zusammenhang geschilderten Vorteile werden in gleicher Weise bei einer korrespondierenden Vorrichtung zur Durchführung des Verfahrens erreicht, so dass hierauf Bezug genommen werden kann. Demgemäß ist es für eine Vorrichtung vorgesehen, dass die Sensoreinheit zur Bestimmung der Konzentration von Lactat und/oder Pyruvat als Referenzsubstanz in der Probenflüssigkeit ausgebildet ist.

Als weitere Variante oder Kombination wird vorgeschlagen, dass die Auswerteeinheit ein Auswerteprogramm aufweist, welches die Wiederfindungsrate ($R_{GLU}$) für Glucose aus einer nichtlinearen Beziehung zu der Wiederfindungsrate ($R_{REF}$) für die ionische Referenzsubstanz bestimmt und den Glucose-Messwert damit korrigiert.

Im Folgenden wird die Erfindung anhand der in der Zeichnung dargestellten Ausführungsbeispiele näher erläutert. Es zeigen

Fig. 1 ein Blockschaltbild einer Messanordnung zur Glucosebestimmung;

Fig. 2 eine schematische Darstellung eines Mikrodialysesystems als Messanordnung;

Fig. 3 eine Sonde des Mikrodialysesystems gemäß Fig. 2 in ausschnittsweise vergrößerter Darstellung;

Fig. 4 ein stark vereinfachtes Reaktionsschema der Glucoseverarbeitung in Körperzellen;

Fig. 5 empirische Messdaten einer Mikrodialyseuntersuchung mit einer Lactat-/Pyruvat-Kompensations-kurve;

Fig. 6 ein Messdiagramm entsprechend Fig. 5 für eine Untersuchung mittels Ultrafiltration; und

Fig. 7 eine Messdiagramm der Glucose- und Natrium-Wiedergewinnung mittels Mikrodialyse mit einer errechneten Kompensationskurve.

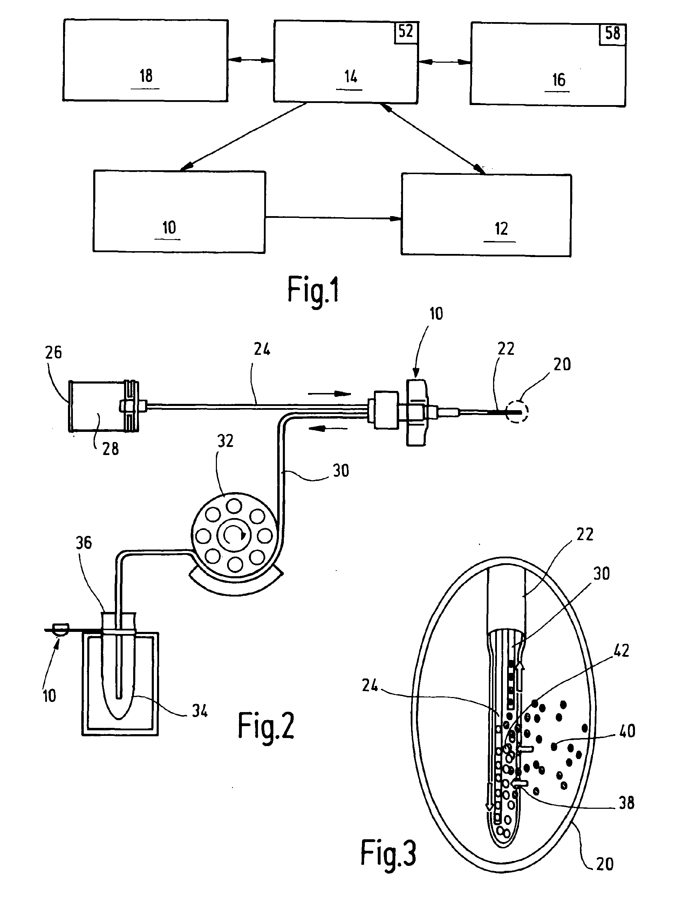

Die Messanordnung gemäß Fig. 1 besteht im Wesentlichen aus einer im subkutanen Gewebe implantierbaren Sonde 10 zur Gewinnung einer Probenflüssigkeit, einer Sensorik 12 zum Nachweis von Inhaltsstoffen in der Probenflüssigkeit, einer Auswerte- und Kontrolleinheit 14 zur Verarbeitung der Sensorsignale und Steuerung des Messablaufs sowie gegebenenfalls einer Ausgabeeinheit 16 und einem Interface 18 zur Anzeige bzw. Weiterleitung der Messergebnisse. Die Sonde 10 kann zur Gewinnung der Probenflüssigkeit in an sich bekannter Weise mit einer Perfusionsflüssigkeit beaufschlagt werden (Mikrodialyse, Mikroperfusion) oder auf Unterdruck gesetzt werden (Ultrafiltration).

In Fig. 2 ist ein Mikrodialysesystem zur kontinuierlichen Probengewinnung und Messdatenerfassung näher veranschaulicht. Die Mikrodialysesonde 10 weist einen im Gewebe 20 befindlichen doppel-lumigen Membrankatheter 22 auf. Dieser ist über eine Zuführleitung 24 aus einem Flüssigkeitsreservoir 26 mit einer Perfusionsflüssigkeit 28 durchspülbar. Die Perfusionsflüssigkeit 28 besteht beispielsweise aus physiologischer Kochsalzlösung. Die Rückflussleitung 30 der Dialysesonde 10 führt über eine Peristaltik- bzw. Rollendosierpumpe 32 zu einem Sammelbehältnis 34. In der Rückflussleitung 30 ist eine im Durchfluss arbeitende Messzelle 36 der Sonde 10 extrakorporal angeordnet. Die Messzelle 36 erfasst beispielsweise über eine elektrochemische Messung die Konzentration der gesuchten Analyten im Dialysat, speziell Glucose und eine Referenz- bzw. Kontrollsubstanz.

Die Funktionsweise der Mikrodialysesonde 10 lässt sich aus Fig. 3 näher ersehen. Der Katheter 22 weist im Bereich seines distalen Endes eine Dialysemembran 38 auf, die in das Gewebe 20 eingebettet ist, so dass durch den Zuflusskanal 24 einströmendes Perfusat durch die semi-permeable Membran hindurch mit Inhaltsstoffen 40 aus dem interzellularen Gewebe befrachtet wird. Die Porosität der Membran 38 ist dabei so bemessen, dass die zu bestimmenden Stoffwechselprodukte, speziell Glucose, Lactat und Pyruvat nahezu widerstandsfrei in das Perfusat gelangen können, während größere Moleküle zurückgehalten werden. Im Unterschied dazu wird bei der Mikroperfusion auf eine Membran verzichtet und der Sondeninnenraum über Wanddurchbrüche direkt an das Gewebe angekoppelt. In beiden Fällen wird die gewonnene Probenflüssigkeit 42 über die gegenüber der Austrittsöffnung der Zuleitung 24 proximal zurückversetzte Mündung des Rückführkanals 30 abgesaugt.

Aufgrund verschiedener Umstände, insbesondere lokalen Veränderungen und Transportwiderständen sowie Perfusionsraten, ist jedoch die Glucosekonzentration im Dialysat bzw. Effusat nicht gleichzusetzen mit dem Glucosegehalt der Gewebeflüssigkeit. Um hier Abhilfe zu schaffen, ist die Sensoreinheit 10 in Verbindung mit der Auswerteeinheit 14 zur zusätzlichen Konzentrationsbestimmung einer endogenen Referenz- bzw. Kontrollsubstanz ausgebildet, um so eine Kompensation von die Messung verfälschenden Effekten durchführen zu können. Als "goldener Standard" wird hierbei die Glucosekonzentration im Kapillarblut angesehen, die mittels bekannter Tests durch Spotmessungen ermittelt werden kann.

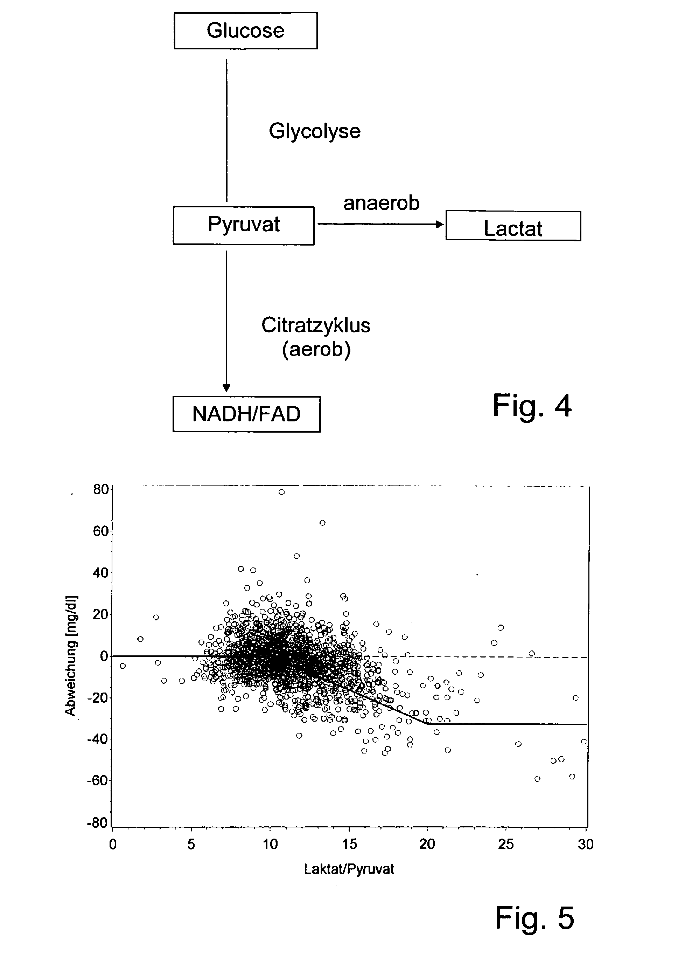

Ein Aspekt betrifft die Korrektur des Messwertes für Glucose anhand des Konzentrationsverhältnisses von Lactat zu Pyruvat in der Probenflüssigkeit. Hintergrund dabei ist die Glucoseverarbeitung in den Gewebezellen, wie in Fig. 4 stark vereinfacht veranschaulicht. Durch Glycolyse wird Glucose zu Pyruvat abgebaut, welches wiederum in einem aeroben Prozess mit hoher Energieausbeute verwertet werden kann, wobei möglichst viele H-Atome (in Form von $NADH/H^+$ und $FADH_2$) gewonnen werden, die mit Sauerstoff zu Wasser reagieren und ATP bilden (Citratzyklus). Unter anaeroben Bedingungen wird in einem konkurrierenden Stoffwechselpfad Pyruvat zu Lactat abgebaut. Dieser Prozess liefert weniger Energie, so dass die Zellen lokal mehr Glucose verarbeiten müssen, um ihren Energiebedarf zu decken. Der nachstehend näher erläuterten Kompensationsmethode liegt nun der Gedanke zugrunde, dass bei einer Lactat-Zunahme ein Ausnahmefall vorliegt, der darauf hindeutet, dass die allgemeine Glucosekonzentration im Körper größer ist als die lokale Konzentration in der Umgebung der Sonde. Solche lokalen Veränderungen könnten beispielsweise durch das Einführen der Sonde oder durch Sondenbewegungen im Gewebe entstehen.

Fig. 5 zeigt ein Messdiagramm in Verbindung mit einer vorgeschlagenen Kompensationskurve. Aufgetragen ist die Abweichung bzw. Differenz der parallel gemessenen Glucosewerte für Probenflüssigkeit und Blut über dem Verhältnis von Lactat zu Pyruvat in der Probenflüssigkeit. Ziel der Kompensation ist es, die Abweichung gegenüber den Werten im Blut zu minimieren. Zu diesem Zweck wird in der Auswerteeinheit eine Korrekturrechnung dahingehend durchgeführt, dass bei einem Konzentrationsverhältnis Lactat/Pyruvat zwischen 10:1 und 20:1 eine lineare Korrektur durchgeführt wird (Kurvenabschnitt 44), und dass ab einem Konzentrationsverhältnis von 20:1 der Messwert für Glucose durch eine Konstante korrigiert wird (Kurvenabschnitt 46). Diese Konstante ergibt sich aus Fig. 5 direkt durch den Betrag des Abszissenwertes des Abschnitts 46.

Bei der Ultrafiltration wird ohne Zuleitung von Perfusionsflüssigkeit an eine Membransonde 10 mittels Saugpumpe oder Saugspritze ein Unterdruck angelegt, wodurch interstitielle Gewebeflüssigkeit in die Saugleitung gelangt und zu einem Sensor 12 transportiert wird. Die semi-permeable Membran des Katheters lässt nur zu, dass kleine Moleküle mit der Flüssigkeit hindurchdiffundieren und somit eine von der Gewebeflüssigkeit getrennte Probenflüssigkeit bilden. Auch hier können verletzungsbedingte Reaktionen im Gewebe stattfinden, die zu lokalen Veränderungen der Glucosekonzentration führen und mittels Lactat/Pyruvat-Messungen in der Probenflüssigkeit korrigiert werden können.

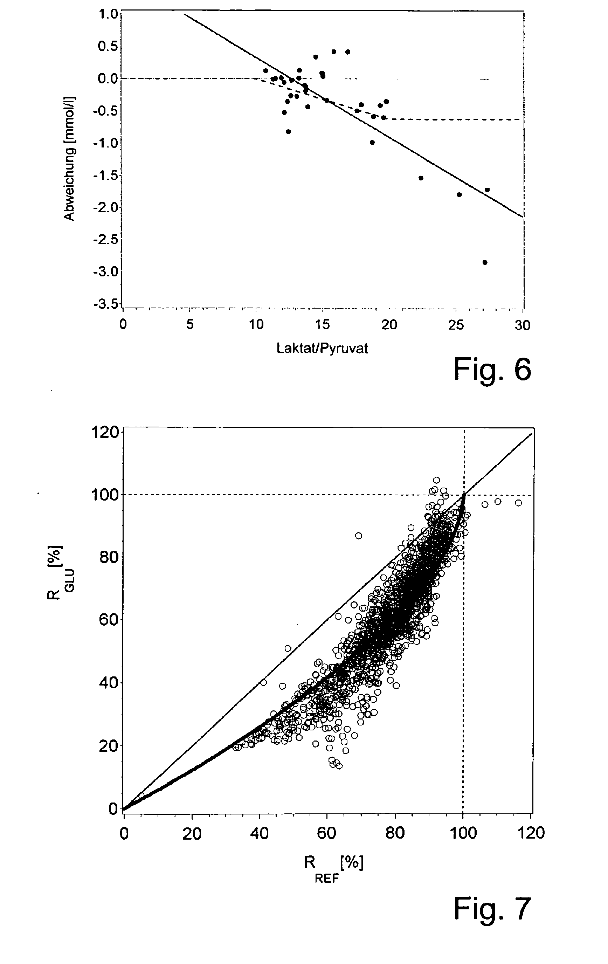

Fig. 6 zeigt mittels Ultrafiltration gewonnene Messwerte, wobei wiederum die Differenz des erfassten Glucosewertes in der Probenflüssigkeit gegenüber Vergleichsmessungen im Blut über dem Verhältnis von Lactat zu Pyruvat als Ordinate aufgetragen ist. Die gestrichelte Korrekturkurve 48 entspricht der oben zu Fig. 5 erläuterten Kompensation. Möglich ist es auch, dass entsprechend der durchgezogenen Linie 50 nur eine lineare Korrektur beispielsweise in einem Konzentrationsbereich zwischen 10:1 und 30:1 Lactat/Pyruvat durchgeführt wird, wobei die Steigung der Kompensationskurve durch eine Anpassung an die Messwerte gewonnen wurde.

Gemäß einem weiteren Erfindungsaspekt werden neben den Glucosewerten in der Probenflüssigkeit auch Messwerte für $Na^+$-Ionen als endogene Referenzsubstanz erfasst. Hierbei wird davon ausgegangen, dass $Na^+$ in der Gewebeflüssigkeit unabhängig von der Glucoseverarbeitung weitgehend konstant bleibt, und dass bei einer simultanen Messung von Glucose und Natriumionen in der Probenflüssigkeit somit Rückschlüsse auf die Transportwiderstände getroffen werden können. Als Wiederfindungsrate wird in diesem Zusammenhang das Konzentrationsverhältnis der jeweiligen Substanz in der Gewebe- und der Probenflüssigkeit definiert. Für eine ionische Referenzkorrektur weist die Auswerteeinheit 14 eine Auswerteroutine 52 auf, welche die Wiederfindungsrate $R_{GLU}$ für Glucose aus einer nichtlinearen Beziehung zu der Wiederfindungsrate $R_{REF}$ für die ionische Referenzsubstanz ($Na^+$) bestimmt und den Glucose-Messwert damit korrigiert.

Speziell ermittelt das Auswerteprogramm 52 die Wiederfindungsrate $R_{GLU}$ für Glucose aus der Beziehung

$$1 - R_{REF} = (1 - R_{GLU})^k \qquad (1)$$

wobei $k$ ein vorgegebener Wert ist.

Die Wiederfindungsrate $R_{REF}$ für die Referenzsubstanz wird als Verhältnis aus dem Messewert für die Referenzsubstanz in der Probenflüssigkeit und einem bekannten konstanten Konzentrationswert in der Gewebeflüssigkeit ermittelt.

Die Grundlage für Gleichung (1) liefert ein nichtlineares Modell für die Wiederfindung bzw. Recovery:

$$Recovery = 1 - \exp\left[-\frac{1}{Q \bullet W_{ges}}\right] \qquad (2)$$

wobei Q die Flussrate der Perfusion und $W_{ges}$ den Gesamtwiederstand als Funktion der Transportwiderstände von Dialysat, Membran und Gewebe bezeichnen.

Aus Gleichung (2) folgt zunächst:

$$\frac{1}{\ln(1 - Recovery)} = -QW_{ges} \qquad (3)$$

d.h. eine logarithmische Transformation führt zu einer linearen Abhängigkeit von der Flussrate.

Gesucht ist nun ein Faktor k, der das Verhältnis der Gesamtwiderstände für Glucose und Natrium für eine bestimmte Dialysemembran kennzeichnet:

$$W_{ges}(Glucose) = k\, W_{ges}(Natrium).$$

Dieser Faktor k kann mit Hilfe von in-vivo-Studien und in-vitro-Daten aus der Gleichung (3) durch Quotientenbildung geschätzt werden.

Der Faktor k ist eine Funktion der Widerstände, also praktisch eine Funktion der Flussrate, Membran und Interstitiumseigenschaften:

$$k = \frac{W_{ges}(Glu)}{W_{ges}(Na)} = \frac{\ln(1 - Recovery(Na))}{\ln(1 - Recovery(Glu))}$$

In-vivo-Studien haben gezeigt, dass auch der Einfluss der Membran und der physiologischen Umgebung (durch osmotische Effekte bestimmt) modellierbar ist und eine zusätzliche Korrektur der Recovery ermöglicht.

Ist k bekannt, so kann der Zusammenhang zwischen Recovery(Glucose) und Recovery(Natrium) aus Gleichung (2) wie folgt hergeleitet werden:

$$1-Recovery(Na) = \exp\left[-\frac{1}{QW_{ges}(Na)}\right]$$

$$= \exp\left[-\frac{k}{QW_{ges}(Glu)}\right]$$

$$= \exp\left[-\frac{1}{QW_{ges}(Glu)}\right]^{k}$$

$$= [1-Recovery(Glu)]^{k} \qquad (1)$$

Fig. 7 zeigt ein Diagramm mit Messwerten der Glucose-Wiedergewinnungsrate über der Na$^{+}$-Wiedergewinnungsrate. Zusätzlich ist ein proportionaler Zusammenhang der Wiedergewinnungsraten (Kurve 54) und die Kompensationskurve 56 nach Gleichung (1) in dem Diagramm dargestellt. Es ist deutlich zu erkennen, dass der proportionale bzw. lineare Verlauf 54 durch die Messwerte nicht bestätigt wird, während die Kompensationskurve 56 vollständig unterhalb der Winkelhalbierenden positiv gekrümmt verläuft und damit die empirischen Messungen zutreffend beschreibt.

Durch die Kompensationskurve 56 kann eine Schwankung der Wiedergewinnung somit ausgeglichen werden, indem ausgehend von einer sich ändernden Na$^+$-Wiedergewinnung auch der Wert für Glucose entsprechend korrigiert wird. Um unterschiedlichen Flussraten Rechnung zu tragen, könnte die Kompensationskurve 56 durch einen zusätzlichen Faktor gestaucht werden, so dass berücksichtigt wird, dass aufgrund eines hohen Durchsatzes keine 100%ige Wiedergewinnung erreicht werden kann.

Zweckmäßig wird Na$^+$ als Referenzsubstanz für eine Korrektur von messtechnischen Abweichungen und Lactat/Pyruvat als Referenzsubstanz für eine Korrektur von lokal bedingten Abweichungen des Messwerts für Glucose in der Probenflüssigkeit von der tatsächlichen Glucose-Konzentration im Gewebe herangezogen.

Eine weitere Verbesserung der Funktionalität kann auch dadurch erreicht werden, dass bei Überschreiten eines Schwellenwerts des Messwerts für die Referenzsubstanz durch einen Signalgeber eine Fehlfunktion der Messanordnung signalisiert wird.

**Patentansprüche**

1. Verfahren zur Bestimmung der Glucose-Konzentration in Gewebeflüssigkeit (40) bei welchem in einer durch Mikrodialyse, Mikroperfusion oder Ultrafiltration aus einem Körpergewebe (20) erhaltenen Probenflüssigkeit (42) Messwerte für Glucose und für eine endogene Referenzsubstanz erfasst werden und der Messwert für Glucose nach Maßgabe des Messwerts für die Referenzsubstanz korrigiert wird, **dadurch gekennzeichnet, dass** die Konzentration von Lactat und/oder Pyruvat als Referenzsubstanz in der Probenflüssigkeit (42) bestimmt wird.

2. Verfahren nach Anspruch 1, **dadurch gekennzeichnet, dass** ein Konzentrationsverhältnis von Lactat zu Pyruvat in der Probenflüssigkeit (42) zur Korrektur des Messwertes für Glucose gebildet wird.

3. Verfahren nach 2, **dadurch gekennzeichnet, dass** bei einem Konzentrationsverhältnis in einem mittleren Bereich (44) vorzugsweise zwischen 10:1 und 20:1 eine lineare Korrektur in Abhängigkeit von dem Konzentrationsverhältnis durchgeführt wird, und in einem höheren Bereich (46) vorzugsweise ab einem Konzentrationsverhältnis von 20:1 der Messwert für Glucose durch eine Konstante korrigiert wird.

4. Verfahren zur Bestimmung der Glucose-Konzentration in Gewebeflüssigkeit (40) bei welchem in einer durch Mikrodialyse oder Mikroperfusion eines Körpergewebes (20) gewonnenen Probenflüssigkeit (42) Messwerte für Glucose und für eine endogene Referenzsubstanz erfasst werden und der Messwert für Glucose nach Maßgabe des Messwerts für die Referenzsubstanz korrigiert wird, **dadurch gekennzeichnet, dass** die Wiederfindungsrate ($R_{GLU}$) für Glucose aus einer nichtlinearen Beziehung zu der Wiederfindungsrate ($R_{REF}$) für die ionische Referenzsubstanz bestimmt und der Glucose-Messwert damit korrigiert wird.

5. Verfahren nach Anspruch 4, **dadurch gekennzeichnet, dass** die Wiederfindungsrate ($R_{GLU}$) für Glucose gemäß der Beziehung

$$1 - R_{REF} = (1 - R_{GLU})^k$$

ermittelt wird, wobei k ein vorgegebener Wert ist.

6. Verfahren nach Anspruch 5, **dadurch gekennzeichnet, dass** der Wert k als Verhältnis der Widerstände für den Übergang von Glucose und Referenzsubstanz zwischen der Gewebe- und der Probenflüssigkeit (40,42) vorzugsweise empirisch bestimmt wird.

7. Verfahren nach einem der Ansprüche 4 bis 6, **dadurch gekennzeichnet, dass** die Wiederfindungsrate ($R_{REF}$) für die Referenzsubstanz als Verhältnis aus dem Messwert für die Referenzsubstanz in der Probenflüssigkeit (42) und einem konstanten Konzentrationswert in der Gewebeflüssigkeit (40) ermittelt wird.

8. Verfahren nach einem der Ansprüche 4 bis 7, **dadurch gekennzeichnet, dass** Natrium (Na$^+$) als körpereigene ionische Referenzsubstanz herangezogen wird.

9. Verfahren nach einem der Ansprüche 1 bis 7, **dadurch gekennzeichnet, dass** Natrium (Na⁺) als Referenzsubstanz für eine Korrektur von messtechnischen Abweichungen und Lactat und/oder Pyruvat als Referenzsubstanz für eine Korrektur von lokal bedingten Abweichungen des Messwerts für Glucose in der Probenflüssigkeit von der tatsächlichen Glucose-Konzentration im Gewebe des Organismus herangezogen wird.

10. Verfahren nach einem der Ansprüche 1 bis 9, **dadurch gekennzeichnet, dass** die Probenflüssigkeit (42) mittels einer im Gewebe befindlichen Sonde (10;22) durch Perfusion mit Spülflüssigkeit oder durch Anlegen eines Unterdrucks gewonnen wird.

11. Verfahren nach einem der Ansprüche 1 bis 10, **dadurch gekennzeichnet, dass** bei Überschreiten einer oberen und/oder unteren vorgegebenen Schwelle des Messwerts für die Referenzsubstanz eine Fehlfunktion signalisiert wird.

12. Verfahren nach einem der Ansprüche 1 bis 11, **dadurch gekennzeichnet, dass** die Messwerte ex vivo erfasst werden.

13. Vorrichtung zur Bestimmung der Glucose-Konzentration in Gewebeflüssigkeit (40) mit einer Sensoreinheit (10) zur Erfassung von Messwerten für Glucose und für eine endogene Referenzsubstanz in einer durch Mikrodialyse, Mikroperfusion oder Ultrafiltration aus einem Körpergewebe (20) erhaltenen Probenflüssigkeit (42) und einer Auswerteeinheit (14) zur Korrektur des Messwerts für Glucose nach Maßgabe des Messwerts für die Referenzsubstanz, **dadurch gekennzeichnet, dass** die Sensoreinheit (10) zur Bestimmung der Konzentration von Lactat und/oder Pyruvat als Referenzsubstanz in der Probenflüssigkeit (42) ausgebildet ist.

14. Vorrichtung nach Anspruch 13, **dadurch gekennzeichnet, dass** die Auswerteeinheit (14) für die Berechnung des Konzentrationsverhältnis von Lactat zu Pyruvat in der Probenflüssigkeit (42) zur Korrektur des Messwertes für Glucose ausgebildet ist.

15. Vorrichtung nach 14, **dadurch gekennzeichnet, dass** die Auswerteeinheit (14) bei einem Konzentrationsverhältnis in einem mittleren Bereich (44) vorzugsweise zwischen 10:1 und 20:1 eine lineare Korrektur in Abhängigkeit von dem Konzentrationsverhältnis durchführt, und darüber den Messwert für Glucose durch eine Konstante korrigiert.

16. Vorrichtung zur Bestimmung der Glucose-Konzentration in Gewebeflüssigkeit (40) mit einer Sensoreinheit zur Erfassung von Messwerten für Glucose und für eine endogene Referenzsubstanz in einer durch Mikrodialyse, Mikroperfusion oder Ultrafiltration aus einem Körpergewebe (20) erhaltenen Probenflüssigkeit (42) und einer Auswerteeinheit (14) zur Korrektur des Messwerts für Glucose nach Maßgabe des Messwerts für die Referenzsubstanz, **dadurch gekennzeichnet, dass** die Auswerteeinheit (14) ein Auswerteprogramm (52) aufweist, welches die Wiederfindungsrate ($R_{GLU}$) für Glucose aus einer nichtlinearen Beziehung zu der Wiederfindungsrate ($R_{REF}$) für die ionische Referenzsubstanz bestimmt und den Glucose-Messwert damit korrigiert.

17. Vorrichtung nach Anspruch 16, **dadurch gekennzeichnet, dass** das Auswerteprogramm (52) die Wiederfindungsrate ($R_{GLU}$) für Glucose aus der Beziehung

$$1 - R_{REF} = (1 - R_{GLU})^k$$

ermittelt, wobei k ein vorgegebener Wert ist.

18. Vorrichtung nach Anspruch 16 oder 17, **dadurch gekennzeichnet, dass** das Auswerteprogramm (52) die Wiederfindungsrate ($R_{REF}$) für die Referenzsubstanz als Verhältnis aus dem Messewert für die Referenzsubstanz in der Probenflüssigkeit (42) und einem konstanten Konzentrationswert in der Gewebeflüssigkeit (40) ermittelt.

19. Vorrichtung nach einem der Ansprüche 16 bis 18, **dadurch gekennzeichnet, dass** bei Überschreiten einer oberen und/oder unteren vorgegebenen Schwelle des Messwerts für die Referenzsubstanz durch einen Signalgeber (58) eine Fehlfunktion signalisiert wird.

**20.** Vorrichtung nach einem der Ansprüche 16 bis 19, **dadurch gekennzeichnet, dass** die körpereigene ionische Referenzsubstanz Natrium ($Na^+$) ist.

Fig.1

Fig.2

Fig.3

Fig. 4

Fig. 5

Fig. 6

Fig. 7

**Europäisches Patentamt**

# EUROPÄISCHER RECHERCHENBERICHT

Nummer der Anmeldung

EP 05 00 9986

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A | EP 0 367 752 A (AVL MEDICAL INSTRUMENTS AG) 9. Mai 1990 (1990-05-09) * Spalte 1, Zeile 1 - Zeile 14 * * Spalte 2, Zeile 54 - Spalte 3, Zeile 24 * * Spalte 6, Zeile 44 - Zeile 55 * * Spalte 10, Zeile 40 - Spalte 11, Zeile 17; Anspruch 1 * ----- | 1-3,9-15 | A61B5/00 |
| A | DE 41 23 441 A1 (AVL MEDICAL INSTRUMENTS AG, SCHAFFHAUSEN, CH) 23. Januar 1992 (1992-01-23) * Spalte 1, Zeile 1 - Spalte 2, Zeile 5; Anspruch 1 * ----- | 1-3,9-15 | |
| A | EP 1 072 222 A (ROCHE DIAGNOSTICS GMBH) 31. Januar 2001 (2001-01-31) * Zusammenfassung * ----- | 1,13 | |
| A | US 5 118 473 A (COLEMAN ET AL) 2. Juni 1992 (1992-06-02) * Zusammenfassung * ----- | 1,13 | |
| A | WO 03/003911 A (SCHAUPP, LUKAS; PIEBER, THOMAS) 16. Januar 2003 (2003-01-16) * Seite 6, Zeile 1 - Seite 10, Zeile 14 * ----- | 4-12, 16-20 | RECHERCHIERTE SACHGEBIETE (IPC) A61B G01N |
| A | EP 0 534 074 A (INSTITUT FUER DIABETESTECHNOLOGIE GEMEINNUETZIGE FORSCHUNGS - UND ENTW) 31. März 1993 (1993-03-31) * Zusammenfassung * ----- | 4,16 | |
| A | EP 0 664 989 A (PFEIFFER, ERNST, PROF. DR; INSTITUT FUER DIABETESTECHNOLOGIE, GEMEINNU) 2. August 1995 (1995-08-02) * Zusammenfassung * ----- | 1,16 | |

Der vorliegende Recherchenbericht wurde für alle Patentansprüche erstellt

| Recherchenort | Abschlußdatum der Recherche | Prüfer |
|---|---|---|
| München | 7. Februar 2006 | Komenda, P |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

...........................................................................
& : Mitglied der gleichen Patentfamilie, übereinstimmendes Dokument

EPO FORM 1503 03.82 (P04C03)

**Europäisches
Patentamt**

Nummer der Anmeldung

EP 05 00 9986

## GEBÜHRENPFLICHTIGE PATENTANSPRÜCHE

Die vorliegende europäische Patentanmeldung enthielt bei ihrer Einreichung mehr als zehn Patentansprüche.

☐ Nur ein Teil der Anspruchsgebühren wurde innerhalb der vorgeschriebenen Frist entrichtet. Der vorliegende europäische Recherchenbericht wurde für die ersten zehn sowie für jene Patentansprüche erstellt, für die Anspruchsgebühren entrichtet wurden, nämlich Patentansprüche:

☐ Keine der Anspruchsgebühren wurde innerhalb der vorgeschriebenen Frist entrichtet. Der vorliegende europäische Recherchenbericht wurde für die ersten zehn Patentansprüche erstellt.

## MANGELNDE EINHEITLICHKEIT DER ERFINDUNG

Nach Auffassung der Recherchenabteilung entspricht die vorliegende europäische Patentanmeldung nicht den Anforderungen an die Einheitlichkeit der Erfindung und enthält mehrere Erfindungen oder Gruppen von Erfindungen, nämlich:

Siehe Ergänzungsblatt B

☒ Alle weiteren Recherchengebühren wurden innerhalb der gesetzten Frist entrichtet. Der vorliegende europäische Recherchenbericht wurde für alle Patentansprüche erstellt.

☐ Da für alle recherchierbaren Ansprüche die Recherche ohne einen Arbeitsaufwand durchgeführt werden konnte, der eine zusätzliche Recherchengebühr gerechtfertigt hätte, hat die Recherchenabteilung nicht zur Zahlung einer solchen Gebühr aufgefordert.

☐ Nur ein Teil der weiteren Recherchengebühren wurde innerhalb der gesetzten Frist entrichtet. Der vorliegende europäische Recherchenbericht wurde für die Teile der Anmeldung erstellt, die sich auf Erfindungen beziehen, für die Recherchengebühren entrichtet worden sind, nämlich Patentansprüche:

☐ Keine der weiteren Recherchengebühren wurde innerhalb der gesetzten Frist entrichtet. Der vorliegende europäische Recherchenbericht wurde für die Teile der Anmeldung erstellt, die sich auf die zuerst in den Patentansprüchen erwähnte Erfindung beziehen, nämlich Patentansprüche:

**Europäisches
Patentamt**

**MANGELNDE EINHEITLICHKEIT
DER ERFINDUNG
ERGÄNZUNGSBLATT B**

Nummer der Anmeldung

EP 05 00 9986

Nach Auffassung der Recherchenabteilung entspricht die vorliegende europäische Patentanmeldung nicht den Anforderungen an die Einheitlichkeit der Erfindung und enthält mehrere Erfindungen oder Gruppen von Erfindungen, nämlich:

1. Ansprüche: 1-3, 9-12 (wenn abhängig von 1-3), 13-15

    Verfahren und Vorrichtung zur Bestimmung der Glukose-Konzentration unter Verwendung von Lactat und Pyruvat.
    ---

2. Ansprüche: 4-8, 9-12 (wenn abhängig von 4-7), 16-20

    Verfahren und Vorrichtung zur Bestimmung der Glukose-Konzentration unter Korrektur des Glukose-Messwertes durch die Wiederfindungsrate.
    ---

EP 1 719 447 A1

## ANHANG ZUM EUROPÄISCHEN RECHERCHENBERICHT
## ÜBER DIE EUROPÄISCHE PATENTANMELDUNG NR.

EP 05 00 9986

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

07-02-2006

| Im Recherchenbericht angeführtes Patentdokument | | | Datum der Veröffentlichung | Mitglied(er) der Patentfamilie | | | Datum der Veröffentlichung |
|---|---|---|---|---|---|---|---|
| EP 0367752 | A | | 09-05-1990 | AR | 245986 | A1 | 30-03-1994 |
| | | | | BR | 8905541 | A | 29-05-1990 |
| | | | | CN | 1042301 | A | 23-05-1990 |
| | | | | DE | 58906306 | D1 | 13-01-1994 |
| | | | | DK | 525889 | A | 01-05-1990 |
| | | | | JP | 1853711 | C | 07-07-1994 |
| | | | | JP | 2177941 | A | 11-07-1990 |
| | | | | JP | 5062543 | B | 08-09-1993 |
| | | | | RU | 2016540 | C1 | 30-07-1994 |
| DE 4123441 | A1 | | 23-01-1992 | AT | 398694 | B | 25-01-1995 |
| | | | | AT | 153090 | A | 15-06-1994 |
| | | | | US | 5243982 | A | 14-09-1993 |
| EP 1072222 | A | | 31-01-2001 | AT | 268141 | T | 15-06-2004 |
| | | | | DE | 19935165 | A1 | 01-02-2001 |
| | | | | JP | 2001066313 | A | 16-03-2001 |
| | | | | US | 6852500 | B1 | 08-02-2005 |
| US 5118473 | A | | 02-06-1992 | KEINE | | | |
| WO 03003911 | A | | 16-01-2003 | AT | 412060 | B | 27-09-2004 |
| | | | | AT | 10572001 | A | 15-02-2004 |
| | | | | EP | 1404217 | A2 | 07-04-2004 |
| | | | | US | 2004168934 | A1 | 02-09-2004 |
| EP 0534074 | A | | 31-03-1993 | AT | 134851 | T | 15-03-1996 |
| | | | | DE | 4130742 | A1 | 18-03-1993 |
| EP 0664989 | A | | 02-08-1995 | DE | 4401400 | A1 | 20-07-1995 |
| | | | | US | 5640954 | A | 24-06-1997 |

EPO FORM P0461

Für nähere Einzelheiten zu diesem Anhang : siehe Amtsblatt des Europäischen Patentamts, Nr.12/82

15